Europäisches Patentamt

European Patent Office

Office européen des brevets

(11) Numéro de publication : **0 380 897 B1**

(12) **FASCICULE DE BREVET EUROPEEN**

(45) Date de publication du fascicule du brevet :
**19.11.92 Bulletin 92/47**

(21) Numéro de dépôt : **89403650.8**

(22) Date de dépôt : **26.12.89**

(51) Int. Cl.⁵ : **A61K 31/57,** A61K 31/435, A61K 31/54, A61K 9/02, // (A61K31/57, 31:435), (A61K31/435, 31:19, 31:415), (A61K31/54, 31:435)

(54) **Nouveaux agents pharmacologiques pour la stérilisation féminine par voie intra-utérine et leur emploi thérapeutique.**

(30) Priorité : **31.01.89 FR 8901205**

(43) Date de publication de la demande :
**08.08.90 Bulletin 90/32**

(45) Mention de la délivrance du brevet :
**19.11.92 Bulletin 92/47**

(84) Etats contractants désignés :
**AT BE CH DE ES FR GB GR IT LI LU NL SE**

(56) Documents cités :
**FR-A- 2 197 582
GB-A- 1 338 493
US-A- 4 158 050**

(73) Titulaire : **Zipper, Jaime
c/o Bernardo Berdichewsky 5465 Manitoba Street
Vancouver, B.C. V5Y 3C3 (CA)**

(72) Inventeur : **Zipper, Jaime
c/o Bernardo Berdichewsky 5465 Manitoba Street
Vancouver, B.C. V5Y 3C3 (CA)**

(74) Mandataire : **Cabinet Pierre HERRBURGER
115, Boulevard Haussmann
F-75008 Paris (FR)**

## Description

La présente invention a pour objet de nouvelles compositions pharmaceutiques destinées à prévenir la grossesse en réalisant une stérilisation non chirurgicale chez la femme, par occlusion chimique des trompes.

Elle a plus particulièrement pour objet des compositions pharmaceutiques améliorées contenant en tant que principe actif de la quinacrine ou un de ses sels d'addition avec un acide minéral ou organique administrable par voie intra-utérine notamment sous forme de comprimés qui provoquent moins d'effets secondaires tout en entraînant une stérilisation plus complète.

Elle a plus spécifiquement pour objet des compositions pharmaceutiques améliorées destinées à réaliser une contraception chimique par occlusion des trompes à l'aide de quinacrine, caractérisées en ce qu'elles contiennent également au moins un adjuvant pour l'action stérilisante de la quinacrine et pour la suppression des effets secondaires gênants déclenchés par celle-ci constitué par un agent anti-inflammatoire et antiprostaglandine choisi dans le groupe formé par les stéroïdes de type cortisonique et les agents anti-inflammatoires non stéroïdiens, cet adjuvant et la quinacrine pouvant être administrés simultanément ou successivement.

Lorsqu'ils sont administrés successivement les deux types de principes actifs peuvent l'être par la même voie et notamment sous forme de comprimés intra-utérins ou par des voies différentes et en particulier l'agent anti-inflammatoire et anti-prostaglandine est administré de préférence par voie parentérale et en particulier par voie intramusculaire, sous forme de solution ou de suspension injectable.

Selon une autre caractéristique de l'invention, la composition pharmaceutique contient un excipient ou un véhicule inerte non toxique et pharmaceutiquement acceptable convenant pour l'administration par voie orale, intra-utérine, intra-musculaire.

Selon une autre caractéristique de l'invention, l'agent anti-inflammatoire et anti-prostaglandine du type cortisonique est choisi dans le groupe constitué notamment par la Dexaméthasone et la Betaméthasone.

Selon une autre caractéristique de l'invention, l'agent anti-inflammatoire et anti-prostaglandine non stéroïdien est choisi dans le groupe constitué notamment par le sel de sodium du Diclofenac, la Dypyrone et le Piroxicam.

Selon une autre caractéristique de l'invention, la quinacrine est employée sous forme de base.

Selon une autre caractéristique de l'invention, la composition renferme une teneur en quinacrine comprise entre 180 et 250 mg par dose unitaire.

Selon une autre caractéristique de l'invention, la teneur en agent anti-inflammatoire et anti-prostaglandine du type cortisonique est comprise entre 3 et 5 mg par dose unitaire.

Selon une autre caractéristique de l'invention, la teneur minimale en agent anti-inflammatoire et anti-prostaglandine de la composition est de 75 mg par dose unitaire.

Selon une autre caractéristique de l'invention, l'agent anti-inflammatoire et anti-prostaglandine est constitué par une préparation liquide administrable par voie parentérale.

Selon une autre caractéristique de l'invention, l'agent anti-inflammatoire et anti-prostaglandine est constitué par un comprimé administrable par voie intra-utérine.

L'invention concerne donc des agents pharmacologiques et adjuvants pour l'action stérilisante de la quinacrine par voie intra-utérine et pour la suppression des effets secondaires gênants déclenchés quelques heures après l'insertion d'un comprimé de quinacrine par voie intra-utérine pour effectuer la stérilisation féminine sans intervention chirurgicale.

### 1 - Bases pour améliorer la technique de stérilisation féminine déjà brevetée -

Le brevet princeps (1) US-A-4 158 080 décrit l'utilisation de la quinacrine comme agent actif, la quinacrine s'étant révélée l'élément le meilleur de toute une famille de dérivés de l'acrioline, identifiée dans le document GB-A-1 338 493 et en particulier dans l'exemple 1 de ce document.

Depuis la publication de ce brevet princeps (1), une liste extensive de travaux de recherches pré-cliniques et cliniques a été publiée (la bibliographie en est incluse) sur la contraception chimique par occlusion des trompes. Néanmoins aucun effet n'a été rapporté dans la littérature quelques heures après l'insertion, ni dans les protocoles de suivi, ni dans une de nos études, qui incluait 143 femmes qui font l'objet de notre étude de Décembre 1979 à octobre 1982 et dans une seconde étude de 112 femmes qui avaient reçu 250 mg de comprimés ayant un temps de dissolution de 100 minutes et recrutées depuis Juillet 1982 jusqu'en Octobre 1984 (Adv.. in Contraception 3 (1987) 255-261).

La seule exception se trouvait dans la publication de Tina Agostina et India Kusma (3) [Evaluation en clinique des comprimés de quinacrine pour la stérilisation féminine chimique] du conseil de coordination du groupe de Recherche Indonésien sur la fertilité (BKS Penfin) présenté au 11ème Congrès AOCOG du 6-12 Décembre 1987 à HONG KONG dans lequel dans le Tableau 14 de ce document, ils divulguent le fait qu'après la première insertion de quinacrine (250 mg), 10 % des sujets ont présenté de la fièvre et 9 % des maux de tête. Après la troisième insertion 2 % ont eu de la fièvre et 10,2 % des maux de tête. Ces effets n'ont pas été rapportés en raison du fait que les visites de suivi avaient été prévues pour le mois suivant de façon à procéder à l'insertion du second comprimé le sujet étant en mesure de revenir plus précocement dans le cas d'un malaise important (beaucoup de sujets n'ont pas considéré comme important un état fébride pendant un ou trois jours ou un mal de tête pendant un ou trois jours). C'est pourquoi ils n'ont pas fait mention de cette survenue qui s'est produite dans ± 10 % des sujets lorsqu'ils venaient pour la seconde insertion.

Nouvelles études expérimentales séries de quinacrine à 250 mg -

Un nouveau protocole pour de nouvelles séries d'expériences dans lesquelles des sujets doivent revenir 24 heures après l'insertion, nous ont montré que 209 sujets qui ont reçu deux insertions de comprimés à 250 mg et qui ont achevé 2544 mois d'observations, ont présenté comme important :

a) des maux de tête qui sont apparus le surlendemain du jour de l'insertion et ont duré pendant de quelques heures à quatre jours. Ce symptôme est survenu chez 6 % des sujets dans les deux insertions ou seulement dans une seule.

b) un phénomène de fièvre de 38°C à 39,5°C apparaissant le surlendemain du jour d'insertion et durant de 1 à 4 jours, sans relation avec le mal de tête. Les deux symptômes sont corrélés chez seulement quelques sujets. La fièvre est apparue chez 5 % des cas (parfois lors de la première insertion et parfois lors de la seconde). Les autres complications ont été semblables à celles décrites dans l'article publié précédemment J. Zipper et al. Adv. in Contraception 3(1987)255. A la fin de l'année de suivi, le taux de grossesse a été de 3,0 pour 100 femmes et par an.

Séries de Quinacrine à 180 mg -

Un autre groupe utilisant la quinacrine seule a été constitué, qui a subi deux insertions intra-utérines avec des comprimés à 180 mg. Les insertions ont été séparées par un cycle menstruel - 190 sujets sont entrés dans cette étude. Pendant les premiers jours, 5,2 % des cas ont montré des maux de tête qui ont duré pendant de quelques heures à quatre jours ; et de la fièvre s'échelonnant de 38°C à 39,5°C qui est survenue le surlendemain du jour d'insertion et a duré pendant une période qui a varié de 1 à 4 jours, sans relation avec les maux de tête. Chez un petit nombre de sujets seulement les deux symptômes ont été présents simultanément. La fièvre est apparue dans 3% des cas (pas nécessairement lors des deux insertions). Ce groupe a manifesté un taux de grossesse de 3 pour 100 femmes, et par an à la fin de la première année du suivi. Les deux groupes quinacrine (250 et 180 mg) ont été considéré comme des témoins pour les autres groupes qui ont reçu un anti-inflammatoire, un antipyrétique et des substances anti-prostagrandines.

Ces complications - fièvre dans 3 et 5 % des cas et maux de tête dans 6 et 5,2 % des cas ont été considérés comme significatifs (c'est pourquoi des médicaments anti-inflammatoires et antipyrétiques sont en cours d'étude afin d'être administré en même temps que les comprimés.

Pour être complets, il convient de mentionner l'existence du document FR-A-2 197 582 qui concerne exclusivement les dispositifs mécaniques intra-utérins anti-conceptionnellement actifs communément nommés stérilets. De tels dispositifs constituent des corps étrangers dont la présence dans l'utérus est de nature à donner naissance à des cellules inflammatoires : les leucocytes qui interfèrent avec le passage du sperme ou de l'ovule de manière à empêcher la conception.

Pour être efficaces, ces dispositifs intra-utérins doivent avoir une taille relativement importante entraînant des effets secondaires indésirables ; on est par suite amené à réduire leur dimension, mais au détriment de l'efficacité.

Pour remédier à cet inconvénient, il est proposé, conformément au document susmentionné, d'incorporer dans le dispositif intra-utérin des agents actifs cytotoxiques tels que par exemple le chlorhydrate de quinacrine susceptible de diffuser lentement au travers du dispositif pour prolonger dans le temps l'effet contraceptif.

Ces agents actifs entraînent également la production de leucocytes qui s'ajoutent à ceux provenant directement de l'action du dispositif pour renforcer l'effet de celui-ci. La concentration de ces agents actifs doit toutefois être suffisamment faible pour éviter les risques de toxicité.

Dans le but de renforcer cette concentration, tout en contrôlant mieux le degré d'inflammation obtenu, il est bien proposé d'associer aux agents actifs un additif autre : l'hydrocortisone, donc un agent anti-inflammatoire stéroïdien de type cortisonien.

Le rôle de cet additif est cependant fondamentalement différent de celui de l'agent actif associé à la quinacrine revendiqué conformément à l'invention et on n'obtient pas le double effet consistant :

- d'une part, à supprimer des états secondaires tels que de la fièvre ou des maux de tête, et

- d'autre part, à renforcer les propriétés stérilisantes.

Il est à cet effet à noter que, à la page 6 du document FR-A-2 197 582, il est mentionné que l'hydrocortisone diminue le nombre de leucocytes que l'on trouve dans le mucosités utérines, ce qui correspond donc à l'inverse du second effet revendiqué dans le cadre de l'invention.

Substances en cours d'étude -

Nous avons décidé d'employer la voie intramusculaire ainsi que la voie intra-utérine pour essayer les substances stéroïdienne anti-inflammatoire.
a) Substances stéroïdiennes anti-inflammatoires
b) Substances anti-inflammatoires non-stéroïdiennes

A - Substances stéroïdiennes anti-inflammatoires -

4 mg de Bétaméthasone et 4 mg de Dexaméthasone ont été employé dans des groupes distincts de sujets.

1 - 4 mg de Betaméthasone intra-musculaire

Ce groupe incluait 90 sujets - 4 mg de Bethaméthasone ont été injectés lorsque le comprimé de quinacrine à 250 mg a été inséré. Ce procédé a été utilisé à nouveau avec le second comprimé (comprimé à 250 mg ayant un temps de dissolution de 100 mn).

Pendant les sept premiers jours de suivi après l'insertion du premier et du second pellet, ni fièvre ni maux de tête n'ont été décelés chez aucun des sujets. Les sujets ont achevé une année de suivi et ont représenté 1220 mois. Seulement une grossesse normotopique a été détectée chez un sujet au sixième mois ordinal.

2 - 4 mg de Betaméthasone intra-utérine (en tant que solution dans 1 ml) -

L'insertion de Betaméthasone intra-utérine a été effectuée simultanément avec l'insertion du comprimé de quinacrine. Ce groupe incluait 65 sujets.

Ni fièvre ni mal de tête n'ont été détectés chez aucun sujet après l'insertion du premier et du second comprimé en même temps que la betamethasone. Ces sujets ont effectué au total 830 mois de suivi. Aucune grossesse n'a été signalée dans ce groupe. Ceci nous a conduit à faire des investigations sur la potentialisation possible des effets occlusifs de la quinacrine chez le rat dû à la betamethasone. Cette méthode expérimentale est celle décrite pour d'autres substances (ref. Agent pharmacologique qui potentialisent ou inhibent l'action occlusivante de la quinacrine, dans le tube du lapin et dans l'utérus de rat cf J. ZIPPER-S.INSUNSA Female Stérilization - pronostic pour des techniques simplifiées pour le sujet ambulatoire Ed Gordon, W. Duncan, R.D. Falb et J. Joseph SPEIDEL (p 131-137), NEW YORK Acad. Press 1972 compte-rendu).

La Betamethasone s'est révélée être active chez le rat, avec des solutions de l'ordre de 005 mg/ml. La base physiologique de cette action n'a pas été déterminée et aucun rapport à ce sujet n'a été publié (cf histologie de l'utérus de rat sous l'influence de 50 mg/ml de quinacrine + 0, 05 mg /ml Betamethasone Figure 1).

III - 4 mg de Dexamethasone Intramusculaire -

Ce groupe inclue 80 sujets. Lorque le comprimé de 250 mg est inséré, 4 mg de Dexamethatone sont injectés par voie intramusculaire. La méthode est réputée avec un second comprimé. Ni maux de tête, ni fièvre n'ont été détectés dans les premiers sept jours de suivi. Les sujets ont achevé 950 mois de suivi. Aucune grossesse n'a été détectée dans ce groupe.

IV - 4 mg de Dexamethasone par voie intra-utérine (sous forme de solution dans 1 ml) -

L'insertion est effectuée après que les comprimé aient été insérés. Ce groupe inclût 60 sujets. Ni fièvre, ni maux de tête n'ont été détectés durant la première semaine après insertion du comprimé. Ce groupe avait 850 mois de suivi. Aucune grossesse n'a été détectée.

CONCLUSION

La bétaméthasone et la Dexamethasone sont des stéroïdes ayant une activité puissante antiphlogistique, anti-inflammatoire et anti-oedemateuse au niveau cérébral et sont des adjuvants importants pour réduire les effets secondaires indésirables de la quinacrine lorsque cette substance est employée pour provoquer l'occlusion la région intra-murale des trompes humaines afin de réaliser une stérilisation pharmacologique. En outre, un mécanisme d'action qui n'est pas clair, semble renforcer l'action d'occlusion de la quinacrine à ce niveau. Le nombre total de sujets a été de 295 et a représenté 3850 mois/femmes d'observation. Seulement une grossesse a été détectée.

B - Substances anti-inflammatoires non stéroïdiennes

1 - Diclofenac sel de sodium -

Cet important agent anti-inflammatoire et inhibiteur de synthèse de prostaglandine a été appliqué en une dose unique intramusculaire de 75 mg, lorsque le comprimé de quinacrine à 180 mg a été inséré. Deux insertions par sujet ont été effectuées, séparées par un cycle menstruel - 78 sujets ont été traités - 702 mois de suivi. Dans la première semaine de suivi, ni fièvre ni mal de tête n'ont été détectés chez aucun des sujets. Jusqu'à la fin de l'étude aucune grossesse n'a été détectée.

2 - 50 mg Diclofenac sel sodium ont été administrés par la voie intra-urétine sous forme de comprimés simultanément avec l'insertion des comprimés de quinacrine (180 mg, 10 minutes temps de dissolution). Ce procédé a été utilisé à nouveau avec le second comprimé séparé par un cycle menstruel. Pendant les sept premiers jours de suivi après l'insertion du premier et du second comprimé ni fièvre ni maux de tête n'ont été décelés chez aucun des sujets. Ce groupe incluait 85 sujets. Les sujets ont achevé 1190 mois de suivi. Aucune grossesse n'a été détectée.

3 - Dipyrone (metamizol sel de sodium). Cet agent antipyrétique anti-inflammatoire a été appliqué en une dose unique intramusculaire de 2 g lorsque le pellet de quinacrine à 180 mg (temps de dissolution 10 minutes) a été inséré. Deux insertions, séparées par un cycle mentruel ont été effectuées - 76 sujets ont été traités, 1064 mois de suivi. Dans la première semaine de suivi, ni fièvre ni mal de tête n'ont été détecté chez aucun des sujets. Jusqu'à la fin de l'étude aucune grossesse n'a été détectée. D'autres agents anti-inflammatoires , antiprostaglandine non stéroïdiens comme le Piroxicam, Indomethacine, Ibuprogene, Naproxen ou d'autres peuvent être utilisés.

La recherche avec quelques uns de ces agents est en développement. De même, la recherche avec les inhibiteurs de synthèse de prostaglandines appliqués par voie intrautérine ou intramusculaire une seule fois est en développement.

## Revendications

1. Composition pharmaceutique destinée à réaliser une stérilisation non chirurgicale chez la femme par occlusion chimique des trompes et contenant, en tant que principe actif de la quinacrine ou un de ses sels d'addition avec un acide minéral ou organique administrable par voie intra-utérine notamment sous forme de comprimés, composition pharmaceutique caractérisée en ce qu'elle contient également au moins un adjuvant pour l'action stérilisante de la quinacrine et pour la suppression des effets secondaires gênants déclenchés par celle-ci constitué par un agent anti-inflammatoire et antiprostaglandine choisi dans le groupe formé par les stéroïdes de type cortisonique et les agents anti-inflammatoires non stéroïdiens, cet adjuvant et la quinacrine pouvant être administrés simultanément ou successivement.

2. Composition pharmaceutique selon la revendication 1, caractérisée en ce qu'elle contient un excipient ou un véhicule inerte non toxique et pharmaceutiquement acceptable convenant pour l'administration par voie orale, intra-utérine, ou intra-musculaire.

3. Composition pharmaceutique selon l'une quelconque des revendications 1 et 2, caractérisée en ce que l'agent anti-inflammatoire et anti-prostaglandine du type cortisonique est choisi dans le groupe constitué notamment par la Dexaméthasone et la Betaméthasone.

4. Composition pharmaceutique selon l'une quelconque des revendications 1 et 2, caractérisée en ce que l'agent anti-inflammatoire et anti-prostaglandine non stéroïdien est choisi dans le groupe constitué notamment par le sel de sodium du Diclofenac, la Dypyrone et le Piroxicam.

5. Composition pharmaceutique selon l'une quelconque des revendications 1 et 2, caractérisée en ce que la quinacrine est employée sous forme de base.

6. Composition pharmaceutique selon la revendication 5, caractérisée en ce qu'elle renferme une teneur en quinacrine comprise entre 180 et 250 mg par dose unitaire.

7. Composition pharmaceutique selon la revendication 3, caractérisée en ce que sa teneur en agent anti-inflammatoire et anti-prostaglandine du type cortisonique est comprise entre 3 et 5 mg par dose unitaire.

8. Composition pharmaceutique selon la revendication 4, caractérisée en ce que sa teneur minimale en agent anti-inflammatoire et anti-prostaglandine est de 75 mg par dose unitaire.

9. Composition pharmaceutique selon l'une quelconque des revendications 1 à 8, caractérisée en ce que l'agent anti-inflammatoire et anti-prostaglandine est constitué par une préparation liquide administrable par voie parentérale.

10. Composition pharmaceutique selon l'une quelconque des revendications 1 à 8, caractérisée en ce que l'agent anti-inflammatoire et anti-prostaglandine est constitué par un comprimé administrable par voie intra-utérine.

## Patentansprüche

1. Pharmazeutische Zusammensetzung zur Sterilisation von Frauen ohne chirurgischen Eingriff durch den chemischen Verschluss des Eileiters, die als natürlichen Wirkstoff das Chinacrin oder eines der Zusatzsalze desselben mit einer mineralischen oder organischen Säure enthält und auf intrauterinem Wege, im wesentlichen in Form von Tabletten, eingenommen wird, ein Pharmazeutikum,

dadurch gekennzeichnet,

dass es auch mindestens einen Zusatzstoff für die sterilisierende Wirkung des Chinacrins und die Ausschaltung der störenden Nebenwirkungen, die durch dasselbe ausgelöst werden, enthält und das aus einem entzündungshemmenden und antiprostaglandinen Wirkstoff besteht, der aus der Gruppe der Kortisonsteroide und der entzündungshemmenden Wirkstoffe ohne Steroide stammt, wobei dieser Wirkstoff und das Chinacrin gleichzeitig oder nacheinander verabreicht werden können.

2. Pharmazeutische Zusammensetzung nach Anspruch 1, dadurch gekennzeichnet, dass sie eine ungiftige und pharmazeutisch annehmbare Grundmasse oder inerten Arzneistoffträger enthält, der sich für eine Verabreichung auf oralem, intrauterinem oder intramuskulärem Wege eignet.

3. Pharmazeutische Zusammensetzung nach Anspruch 1 und 2, dadurch gekennzeichnet, dass der kortisonartige entzündungshemmende und antiprostaglandine Wirkstoff aus einer Gruppe gewählt wird, die im wesentlichen aus Dexamethasone und Betamethasone besteht.

4. Pharmazeutische Zusammensetzung nach Anspruch 1 und 2, dadurch gekennzeichnet, dass der entzündungshemmende und antiprostaglandine Wirkstoff ohne Steroide aus einer Gruppe ausgewählt wird, die im wesentlichen aus den Natriumsalzen des Diclofenac, aus Dypyron und Piroxicam besteht.

5. Pharmazeutische Zusammensetzung nach Anspruch 1 und 2, dadurch gekennzeichnet, dass das Chinacrin als Grundstoff verwendet wird.

6. Pharmazeutische Zusammensetzung nach Anspruch 5, dadurch gekennzeichnet, dass ein Chinacrin-Gehalt zwischen 180 und 250 mg je Einheitsdosis enthalten ist.

7. Pharmazeutische Zusammensetzung nach Anspruch 3, dadurch gekennzeichnet, dass der Gehalt an dem entzündungshemmenden und antiprostaglandinen Wirkstoff je nach Art des Kortisons zwischen 3 und 5 mg je Einheitsdosis beträgt.

8. Pharmazeutische Zusammensetzung nach Anspruch 4, dadurch gekennzeichnet, dass der Mindestgehalt an entzündungshemmendem und antiprostaglandinem Wirkstoff 75 mg je Einheitsdosis beträgt.

9. Pharmazeutische Zusammensetzung nach einem der Ansprüche 1 bis 8, dadurch gekennzeichnet, dass der entzündungshemmende und antiprostaglandine Wirkstoff aus einem flüssigen Präparat besteht, das auf parenteralem Wege verabreicht werden kann.

10. Pharmazeutische Zusammensetzung nach einem der Ansprüche 1 bis 8, dadurch gekennzeichnet, dass der entzündungshemmende und antiprostaglandine Wirkstoff aus einer Tablette besteht, die auf intrauterinem Wege verabreicht werden kann.

**Claims**

1. Pharmaceutical composition intended to effect non-surgical sterilisation in women by chemical occlusion of the tubes and containing, as the active constituent, quinacrine or one of its addition salts with a mineral or organic acid administrable by the intra-uterine route, particularly in the form of tablets, the pharmaceutical composition being characterised in that it also contains at least one adjuvant for the sterilising action of the quinacrine and for the suppression of troublesome secondary effects triggered by the latter, consisting of an anti-inflammatory and anti-prostaglandin agent chosen from the group formed by steroids of the cortisone type and non-steroid anti-inflammatory agents, this adjuvant and the quinacrine being able to be administered simultaneously or successively.

2. Pharmaceutical composition according to claim 1, characterised in that it contains an excipient or an inert, non-toxic and pharmaceutically acceptable vehicle suitable for administration by the oral, intra-uterine or intra-muscular route.

3. Pharmaceutical composition according to either one of claims 1 and 2, characterised in that the anti-inflammatory and anti-prostaglandin agent of the cortisone type is chosen from the group consisting particularly of Dexamethasone and Betamethasone.

4. Pharmaceutical composition according to either one of claims 1 and 2, characterised in that the non-steroid anti-inflammatory and anti-prostaglandin agent is chosen from the group consisting particularly of Diclofenac sodium salt, Dypyrone and Piroxicam.

5. Pharmaceutical composition according to either one of claims 1 and 2, characterised in that the quinacrine is used in the form of a base.

6. Pharmaceutical composition according to claim 5, characterised in that it has a quinacrine content of between 180 and 250 mg per unit dose.

7. Pharmaceutical composition according to claim 3, characterised in that its anti-inflammatory and anti-prostaglandin agent content, of the cortisone type, is between 3 and 5 mg per unit dose.

8. Pharmaceutical composition according to claim 4, characterised in that its minimum anti-inflammatory and anti-prostaglandin agent content is 75 mg per unit dose.

9. Pharmaceutical composition according to any one of claims 1 to 8, characterised in that the anti-inflammatory and anti-prostaglandin agent consists of a liquid preparation which can be administered parenterally.

10. Pharmaceutical composition according to any one of claims 1 to 8, characterised in that the anti-inflammatory and anti-prostaglandin agent consists of a tablet which can be administered by the intra-uterine route.